(19) Europäisches Patentamt
European Patent Office
Office européen des brevets

(11) Publication number: **0 101 234 B1**

(12) # EUROPEAN PATENT SPECIFICATION

(45) Date of publication of patent specification: **29.10.86**

(51) Int. Cl.[4]: **C 07 D 337/12,** A 61 K 31/38

(21) Application number: **83304420.9**

(22) Date of filing: **01.08.83**

(54) **Therapeutic agent.**

(30) Priority: **06.08.82 GB 8222677**

(43) Date of publication of application:
**22.02.84 Bulletin 84/08**

(45) Publication of the grant of the patent:
**29.10.86 Bulletin 86/44**

(84) Designated Contracting States:
**BE DE FR IT NL SE**

(56) References cited:
**GB-A- 950 717**
**GB-A-1 013 574**
**GB-A-1 226 249**
**GB-A-1 369 525**

(73) Proprietor: **THE BOOTS COMPANY PLC**
**1 Thane Road West**
**Nottingham NG2 3AA (GB)**

(72) Inventor: **Buckett, William Roger**
**27 Trevor Road**
**West Bridgeford Nottingham Notts (GB)**

(74) Representative: **Thacker, Michael Anthony et al**
**THE BOOTS COMPANY PLC Patents Section R4**
**Pennyfoot Street**
**Nottingham NG2 3AA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a novel therapeutic agent, to pharmaceutical compositions containing the novel therapeutic agent and to a process for the manufacture thereof.

The present invention comprises 11-(3-dimethylaminopropylidene)-6,11-dihydrodibenzo(b,e)thiepin-2-carbonitrile of formula I

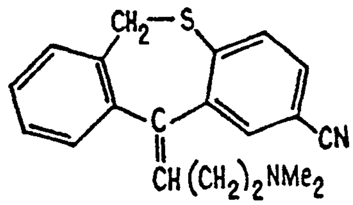

I

and pharmaceutically acceptable salts thereof.

This compound and its salts have been shown to inhibit the uptake of neurotransmitter amines and therefore to have utility in the treatment of depression.

Examples of pharmaceutically acceptable salts are hydrohalides (such as hydrochlorides and hydrobromides), sulphates, phosphates, citrates, tartrates, maleates and fumarates.

The compound of formula I may be prepared from a compound of formula II

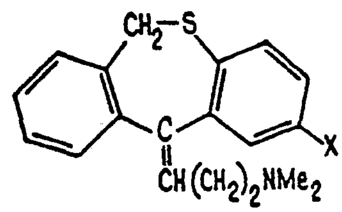

II

a) when X is a leaving group, for example chloro, bromo or iodo by reaction with cuprous cyanide,

b) when X is an iodo group by reaction with potassium cyanide in the presence of tetrakis(triphenylphosphine)palladium(O),

c) when X is an amide group of formula $-CONH_2$ by dehydration with for example thionyl chloride, phosphoryl chloride triphenylphosphine in tetrachloromethane or *p*-toluenesulphonylmethyl isocyanide (sold under the trade name TosMIC) or

d) when X is a group of formula $-CH=NOH$, by dehydration with, for example, phosphoryl chloride, dicyclohexylcarbodiimide in the presence of cupric ion or 1,1'-carbonyldiimidazole.

e) when X is an $NO_2$ group by reduction (for example by stannous chloride) to give an amine group which is converted into a cyano group by means of a Sandmeyer reaction.

Alternatively the compound of formula I may be prepared by the reaction of a compound of formula III

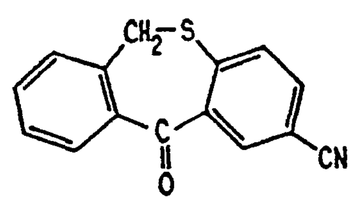

III

with a reagent of formula $Ph_3P=CH(CH_2)_2R$ where R is $NMe_2$ or a group such as a halo group which can be replaced by $NMe_2$.

Compounds of formula I may be prepared by the reaction of the carboxylic acid of formula IV

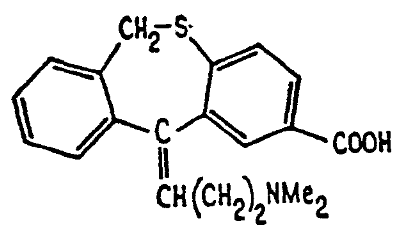

IV

a) with benzenesulphonamide or

b) with urea in the presence of sulphamic acid

The preparation of compounds of formula II in which X is a halo group is described in British Patent Specification 1,013,574. The compound of formula II in which X=$CONH_2$ may be prepared from the corresponding carboxylic acid of formula IV by known methods. The compound of formula II in which X=—CH=NOH may be prepared from the aldehyde of formula V

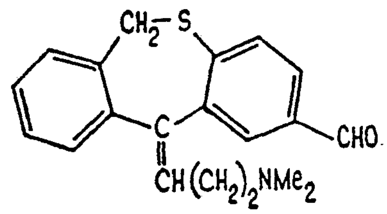

V

by reaction with hydroxylamine.

The compound of formula II in which X is a nitro group may be prepared by methods similar to those described in British Patent Specification 950717.

The compound of formula III may be prepared from the corresponding halo compounds, for example by reaction with cuprous cyanide.

The compound of formula IV may be prepared by the reaction of the known carboxylic acid VI

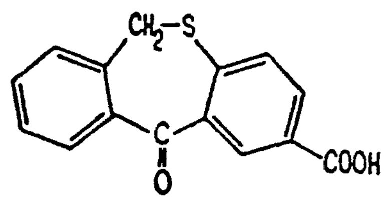

VI

by

a) the reaction of a Grignard reagent for example of formula $Me_2N(CH_2)_2CH_2MgBr$ and

b) the dehydration of the resulting alcohol for example by the use of dilute mineral acids, acetyl chloride or iodine.

The compound of formula V may be prepared by (a) converting the carboxylic acid of formula IV to its acid chloride for example by reaction with thionyl chloride and (b) the reduction of the acid chloride with, for example, lithium tri-*tert*-butoxyaluminohydride at low temperature (e.g. −78°C).

The compound of formula I has a carbon-carbon double bond and therefore can exist in two isomeric forms. The present invention includes both these isomeric forms and mixtures thereof.

The present invention includes pharmaceutical compositions containing a therapeutically effective amount of a compound of formula I together with a pharmaceutically acceptable diluent or carrier.

In therapeutic use, the active compound may be administered orally, rectally, parenterally or topically, preferably orally. Thus the therapeutic compositions of the present invention may take the form of any of the known pharmaceutical compositions for oral, rectal, parenteral or topical administration. Pharmaceutically acceptable carriers suitable for use in such compositions are well known in the art of pharmacy. The compositions of the invention may contain 1—99% by weight of active compound. The compositions of the invention are generally prepared in unit dosage form.

Compositions for oral administration are the preferred compositions of the invention and these are the known pharmaceutical forms for such administration, for example, tablets, capsules, syrups and aqueous or oily suspensions. The excipients used in the preparation of these compositions are the excipients known in the pharmacists' art. Tablets may be prepared by mixing the active compound with an inert diluent such as calcium phosphate in the presence of disintegrating agents, for example, maize starch, and lubricating agents, for example magnesium stearate, and tabletting the mixture by known methods. The tablets may be formulated in a manner known to those skilled in the art so as to give a sustained release of the active compound. Such tablets may, if desired, be provided with enteric coatings by known methods, for example, by the use of cellulose acetate phthalate. Similarly, capsules, for example, hard or soft gelatin capsules, containing the active compound with or without added excipients, may be prepared by conventional means and, if desired, provided with enteric coatings in a known manner. The tablets and capsules may conveniently each contain 1 to 250 mg of the active compound. Other compositions for oral administration include, for example, aqueous suspensions containing the active compound in an aqueous medium in the presence of a non-toxic suspending agent such as sodium carboxymethylcellulose, and oily suspensions containing a compound of the present invention in a suitable vegetable oil, for example arachis oil.

Compositions of the invention suitable for rectal administration are the known pharmaceutical forms for such administration, for example suppositories with cocoa butter or polyethylene glycol bases.

Compositions of the invention suitable for parenteral administration are the known pharmaceutical forms for such administration, for example sterile suspensions in aqueous and oily media or sterile solutions in a suitable solvent.

Compositions for topical administration may comprise a matrix in which the pharmacologically active compound of the present invention are dispersed so that when the matrix is placed in contact with the skin, the compound is administered transdermally. Alternatively the active compound may be dispersed in a pharmaceutically acceptable cream or ointment base.

In the compositions of the present invention the active compound may, if desired, be associated with other compatible pharmacologically active ingredients.

The pharmaceutical compositions containing a therapeutically effective amount of a compound of formula I may be used to treat depression. In such treatment the amount of the compound of formula I administered per day is in the range 1 to 500 mg preferably 3 to 200 mg.

The invention is illustrated by the following description of the preparation of the hydrobromide salt of the compound of formula I (Example 1) and of a structurally related compound (Example A).

Example 1

A mixture of 2-bromo-11-(3-dimethylaminopropylidene)-6,11-dihydrodibenzo(b,e)thiepin (4.2 g prepared in a similar manner to that described in British Patent Specification 1,013,574), cuprous cyanide (1.2 g) and dimethylformamide (5 ml) was heated under reflux for four hours. The mixture was then cooled to around 90°C and poured into a solution of ethylenediamine (2.2 ml) in water (6.6 ml). The resulting suspension was extracted with dichloromethane. The extract was washed with aqueous ethylenediamine solution (8% v/v) and water and then dried and evaporated to give a tarry residue. The residue was dissolved in dichloromethane and applied to chromatograph column (31 × 3 cm) containing alumina (150 g Brockmann Grade I). A section of the alumina (16 to 24 cm from the bottom of the column) was repeatedly extracted with a mixture of 99 parts dichloromethane and 1 part ethanol. Removal of the solvent yielded a residue which was dissolved in dichloromethane (20 ml). The resulting solution was washed with 1N sodium hydroxide solution (20 ml) and water (2 × 20 ml), dried over sodium sulphate and evaporated to dryness to give a residue which was dissolved in ethanol (7 ml). The alcoholic solution was filtered through a diatomaceous earth sold under the Registered Trade Mark CELITE and a mixture of 48% hydrobromic acid and ethanol (4 ml) added to give a pH of 1. The mixture was filtered and evaporated to dryness under reduced pressure. Trituration of the residue with a mixture of ethanol and ether gave 11-(3-dimethylamino-propylidene)-6,11-dihydrodibenzo(b,e)thiepin-2-carbonitrile hydrobromide as a pale brown solid m.p. 213—215°C (decomp.).

Example A

A solution of sodium ethoxide [freshly prepared from sodium metal (3.0 g) and absolute alcohol (60 ml)] was warmed on a steam-bath under reflux and benzenethiol (14.5 g) was added gradually. 6-Bromo-phthalide (28.1 g) was added portionwise with stirring under reflux. Stirring and refluxing were continued for 6 hours.

The cooled reaction mixture was diluted with water (150 ml) and acidified to pH 1 with concentrated hydrochloric acid. The precipitate was collected, washed with water and then re-suspended in water (*ca* 100 ml). Sodium carbonate solution (3N, *ca* 80 ml) was added gradually with stirring and grinding until a pH of 8—9 was attained. The mixture was filtered and the filtrate washed with dichloromethane (4 × 50 ml). Acidification of the aqueous layer to pH 1 with concentrated hydrochloric acid gave 5-bromo-2-phenylthiomethyl-benzoic acid (m.p. 143—145°C) as a pale cream coloured precipitate which was filtered off, washed with water and dried *in vacuo*.

Tetraphosphoric acid (283 g) was heated to 80°C and 5-bromo-2-phenylthiomethylbenzoic acid (34.4 g) was added at this temperature with vigorous stirring. The mixture was heated strongly on the steam-bath (internal temperature *ca* 97°) with stirring for four hours. Ice water (500 ml) was added gradually to the cooled mixture with stirring and the mixture was extracted with dichloromethane (2 × 200 ml). The combined dichloromethane layers were washed with water, aqueous sodium hydroxide solution and water. The dried organic layer was evaporated and the residue crystallised from propan-2-ol to give 9-bromodibenzo[b,e]thiepin-11(6H)-one (m.p. 91—93°C).

A portion of a solution of 3-dimethylaminopropyl chloride (4.5 g) in dry tetrahydrofuran (30 ml) was added under nitrogen to magnesium turnings (0.88 g) in sufficient amount to cover the turnings. Methyl iodide (4 drops) and a crystal of iodine was added initiate the reaction, and the mixture was warmed to boiling point. Refluxing and stirring were continued while the remainder of the tetrahydrofuran solution was added gradually. The mixture was stirred and heated under reflux until the magnesium had almost entirely disappeared.

The mixture was cooled and a solution of the dibenzothiepin-11-one prepared as described above (8.4 g) in dry tetrahydrofuran (40 ml) was added dropwise at 14—17°C. The mixture was gradually warmed and finally stirred and heated at 50°C for 1 hour. The orange solution was concentrated to about half volume under reduced pressure, cooled to 15°C and poured into a solution of ammonium chloride (8.2 g) in water (35 ml). After being collected the precipitate was washed with water, dried *in vacuo*, triturated with petrol (b.p. 40—60°) and again filtered off to give 9-bromo-11-(3-dimethylamino-propyl)-6,11-dihydro-dibenzo[b,e]thiepin-11-ol (m.p. 139—141°).

The above thiepinol (9 g) was mixed with a solution of concentrated sulphuric acid (6.7 ml) in water (75 ml) and boiled under reflux for one and three quarter hours. The mixture was cooled and basified with an

excess of 10*N* aqueous sodium hydroxide solution, and the precipitate was extracted into dichloromethane. After being washed with water the extract was dried and evaporated to give a brown oily residue (8.5 g). This was dissolved in ethanol (25 ml) and acidified to pH 1 using a mixture of 48% HBr (8.5 ml) and ethanol (30 ml), cooling to avoid any undue rise in temperature. The solution was evaporated to dryness under reduced pressure and the residue triturated with alcohol-ether. The product was filtered off and dried *in vacuo* to give 9-bromo-11-(3-dimethylaminopropylidene)-6,11-dihydrobenzo[b,e]thiepin hydrobromide (m.p. 170—172°C (dec.)).

A portion of the hydrobromide (*ca* 8.2 g) was converted into the free base by treatment with water (80 ml) followed by an excess of 2*N* sodium hydroxide solution (15 ml). The sticky precipitate was extracted into dichloromethane and the organic layer was washed with *N* sodium hydroxide solution (30 ml) followed by water (2 × 100 ml). Evaporation of the dried dichloromethane solution gave the base as a brown oily residue (6.5 g).

A mixture of the free base obtained from 9-bromodothiepin hydrobromide as described above, cuprous cyanide (2.3 g) and dry dimethyl formamide (15 ml) was heated at 150°C under reflux for six and a quarter hours. The reaction mixture was allowed to cool and poured into a mixture of dichloromethane (50 ml), water (30 ml) and ethylenediamine (10 ml). The aqueous layer was extracted with further amounts of $CH_2Cl_2$ (3 × 50 ml), and the combined dichloromethane layers were washed repeatedly with 25% v/v aqueous ethylenediamine solution until the aqueous layer ceased to show a blue colouration. The organic layer was washed with water (3 × 50 ml), dried and evaporated to dryness. The dark brown viscous residue (5.8 g) was chromatographed on a column (3.2 × 20 cm) of neutral alumina (Brockmann I, 150 g), developed with dichloromethane. Those portions of the column containing the required product were extracted with dichloromethane containing a little alcohol and the extracts were combined and evaporated. The residue was dissolved in alcohol and made acid to pH paper with HBr. Evaporation to dryness gave a glassy residue which became granular on trituration with a mixture of ether and ethanol. 11-(3-Dimethylaminopropylidene)6,11-dihydrodibenzo[b,e]thiepin-9-carbonitrile hydrobromide (m.p. 180—182°C) was collected and dried.

The biological activity of the compound of formula I has been demonstrated by the following experiments:—

The first of these is an *in vitro* investigation of the inhibition of the uptake of the amines, 5-hydroxytryptamine and noradrenaline. In the experiment the cerebral cortex is removed from adult rats (CD strain) weighing 180 to 200 g and is dissected and chopped into prism shaped slices using a McIlwain tissue chopper cutting in two directions at 45° and at 0.1 mm intervals. The slices are dispersed in chilled Krebs solution (5 ml/g) and samples (0.1 ml) of the suspension containing the equivalent of 20 mg of the cerebral cortex transferred to flasks containing ice-cold Krebs solution (4.3 ml). The flasks were filled with a mixture of oxygen (95%) and carbon dioxide (5%) and sealed. After 15 minutes at 27°C the compound under test and the appropriate amine were added in distilled water (total additional volume 0.6 ml). The amines used were labelled with $^{14}C$ and were

a) 5-hydroxy[side chain-2-$^{14}C$]tryptamine creatinine sulphate hereafter referred to as 5HT, and

b) DL-[carbonol-$^{14}$]noradrenaline DL-bitartrate hereafter referred to as NA.

The flasks were incubated at 27°C for twenty minutes and then the slices collected by filtration. The filter papers with the slices were transferred to scintillation vials containing a scintillation fluid (10 ml) and a mixture (4 ml) of ethanol (3 parts by volume) and methanol (1 part by volume). After the slices had dissolved (at least 45 minutes) the radioactivity was determined quantitatively using a scintillation counter. Control experiments containing no drug were conducted simultaneously with the above experiments and the percentage inhibition of amine uptake ($I_a$) calculated by the equation.

$$I_a = \frac{(\text{Count of control}) - (\text{Count of experiment}) \times 100}{(\text{Count of Control}) - (\text{Background Count})}$$

Background radiation was determined by incubation of controls at 0°C.

The experiments were repeated at three different amine concentrations and the molar concentration of the compound producing a 50% inhibition of amine uptake (IC 50) was determined by linear regression analysis.

In the above test the following results were obtained for the compound of formula I prepared as described in Example 1, the compound of Example A, dothiepin and three other commercially available antidepressants

| Compound | IC50 for 5HT × $10^{-6}$M | IC50 for NA × $10^{-6}$M | IC50 for 5HT / IC50 for NA |
|---|---|---|---|
| Product of Example 1 | 2.1 | 11 | 0.19 |
| Product of Example A | 1.3 | 6.2 | 0.21 |
| Dothiepin | 4.6 | 1.5 | 3.1 |
| Imipramine | 2.1 | 0.7 | 3.0 |
| Clomipramine | 0.6 | 1.6 | 0.38 |
| Amitriptyline | 2.1 | 0.9 | 2.33 |

The effect of the compound prepared in Example 1 on uptake systems for 5HT in the brain was also studied "in vivo" by the following method which involves the use of p-chloroamphetamine as a depletor agent which enters the neurons of the central nervous system via the amine uptake sites. Once inside the neuron the depletor agent displaces the amine transmitter from its storage sites into the cytoplasm of the cell where it is metabolised by monoamine oxidase. This results in a reduction of the levels of the amine transmitter which can be detected experimentally. The levels of the amine transmitter are preserved by pretreatment of the uptake sites with an uptake inhibitor. In the experiments reported below Charles River CD rats were dosed orally with a suspension of the comound under test in a 0.25% solution of hydroxyethyl cellulose (sold under the trade name Cellusolve) in water at a dose of 30 mg/kg. After 30 minutes a solution of p-chloroamphetamine in distilled water was injected intraperitoneally at a dose of 5 mg/kg. After a further four hours the animals were sacrificed and the 5HT level in the brain determined fluorometrically.

The 5HT level in the brain was also determined in Control animals which received only vehicle solutions and for animals which received vehicle orally instead of the test compound followed by intraperitoneal injections of the depleting agent. These latter animals are referred to herein as depleted controls. The percentage inhibition of depletion ($I_b$) was calculated using the formula

$$I_b = \frac{[5HT]\ \text{test animals} - [5HT]\ \text{depleted controls} \times 100}{[5HT]\ \text{controls} - [5HT]\ \text{depleted controls}}$$

In the above test the following values of $I_b$ were obtained

| | |
|---|---|
| Product of Example I | 82% |
| Product of Example A | 0% |
| dothiepin | 9% |
| imipramine | 18% |
| clomipramine | 8% |

These experiments show that the product of Example 1 shows a much greater inhibition of depletion than is shown by the structurally related product of Example A and dothiepin and the two commercially available antidepressants also tested.

It has been proposed that blood platlets act in many ways like brain synaptosomes and have been used for example by Lenehan, Omer and Darragh Arch. int. Pharmacodyn *249* 147—152 (1981)] as a model for CNS serotoninergic neurons. The product of Example 1, the product of Example A and dothiepin have been examined in the following experiments.

Whole blood was collected from both humans and rats and samples (10 ml) were diluted with an anticoagulant solution (1.5 ml) containing citric acid (8 mg $ml^{-1}$), sodium citrate (22 mg $ml^{-1}$), and glucose (24.5 mg ml). The diluted samples were centrifuged at 4°C, 150 g for 20 minutes and the supernatant containing the platelet-rich plasma collected and diluted with an equal volume of 0.9% saline solution.

Tubes containing oxygenated Kreb's solution (4.7 ml) test solution (0.1 ml) and $^{14}$C-radiolabelled 5-hydroxytryptamine (0.1 ml) (final concentration 3.4 × $10^{-8}$M) were placed in a water bath maintained at 37°C. After 10 minutes, a sample of the platelet rich plasma solution was added to each tube and the incubation continued for 5 minutes. The contents of the tubes were then filtered under vacuum through

membrane filters (0.22 μm) and the filters washed with Kreb's solution (3 × 4 ml). The filters were subsequently placed in scintillation fluid (10 ml) and the radioactivity assessed by liquid scintillation spectroscopy. Control samples containing no compound under test and background samples (at 0°C) were run simultaneously so that percentage inhibition $I_c$ of 5-HT uptake could be calculated using the equation

$$I_c = \frac{(\text{Count of Control}) - (\text{Count of test}) \times 100}{(\text{Count of Control}) - (\text{Background Control})}$$

The results using several concentrations of test compound were obtained and the molar concentration of the test compound producing a 50% inhibition of uptake into the platelets (IC50) was determined graphically.

The following results were obtained:—

| | IC50 human platelets (M) | IC50 rat platelets (M) |
|---|---|---|
| product of Example 1 | $1.1 \times 10^{-9}$ | $2.0 \times 10^{-7}$ |
| product of Example A | $1.3 \times 10^{-8}$ | $3.5 \times 10^{-7}$ |
| dothiepin | $2.4 \times 10^{-8}$ | $1.5 \times 10^{-7}$ |
| imipramine (1) | $1 \times 10^{-8}$ | |
| clomipramine (1) | $4.5 \times 10^{-9}$ | |
| amitriptyline (1) | $3 \times 10^{-8}$ | |

(1) Figures obtained from the literature [Lenehan, Omer and Darragh Arch. int. Pharmacodyn *249* 147—152 (1981)].

These results illustrate that the product of Example 1 is more effective at inhibiting uptake into human platelets than into rat platelets and that in human platelets it is more effective than the other compounds tested.

**Claims**

1. 11-(3-dimethylaminopropylidene)-6,11-dihydrodibenzo(b,e)thiepin-2-carbonitrile of formula I

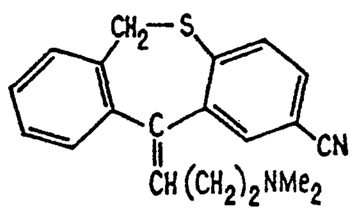

I

and pharmaceutically acceptable salts thereof.

2. 11-(3-dimethylaminopropylidene)-6,11-dihydrodibenzo(b,e)thiepin-2-carbonitrile hydrobromide.

3. Pharmaceutical compositions containing a therapeutically effective amount of the compound claimed in claim 1 or claim 2 together with a pharmaceutically acceptable diluent or carrier.

4. A process for preparing the compound of formula I as claimed in claim 1 from a compound of formula II

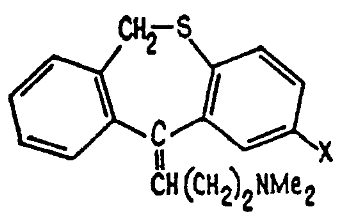

II

a) when X is a leaving group, for example chloro, bromo or iodo by reaction with cuprous cyanide,

b) when X is an iodo group by reaction with potassium cyanide in the presence of tetrakis(triphenylphosphine)palladium(O),

c) when X is an amide group of formula —$CONH_2$ by dehydration with for example phosphoryl chloride triphenylphosphine in tetrachloromethane or *p*-toluenesulphonylmethyl isocyanide.

d) when X is a group of formula —CH=NOH, by dehydration with, for example, phosphoryl chloride, dicyclohexylcarbodiimide in the presence of cupric ion or 1,1′-carbonyldiimidazole.

e) when X is an $NO_2$ group by reduction (for example by stannous chloride) to give an amine group which is converted into a cyano group by means of a Sandmeyer reaction.

5. A process for preparing the compound of formula I as claimed in claim 1 comprising the reaction of a compound of formula III

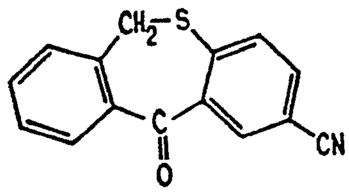

III

with a reagent of formula $Ph_3P{=}CH(CH_2)_2R$ where R is $NMe_2$ or a group such as a halo group which can be replaced by $NMe_2$.

6. A process for preparing the compounds formula I as claimed in claim 1 by the reaction of the carboxylic acid of formula IV

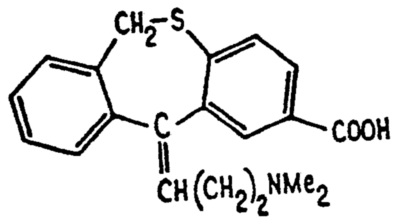

IV

a) with benzenesulphonamide or

b) with urea in the presence of sulphamic acid.

**Patentansprüche**

1. 11-(3-Dimethylaminopropyliden)-6,11-dihydrodibenzo(b,e)thiepin-2-carbonitril der Formel I

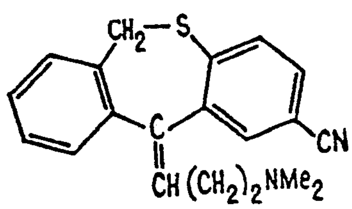

I

und die pharmazeutisch verträglichen Salze davon.

2. 11-(3-Dimethylaminopropyliden)-6,11-dihydrodibenzo(b,e)thiepin-2-carbonitril-hydrobromid.

3. Pharmazeutische Zusammensetzung, enthaltend eine therapeutisch wirksame Menge der Verbindung nach Anspruch 1 oder 2 in Verbindung mit einem pharmazeutisch verträglichen Verdünnungsmittel oder Trägermittel.

4. Verfahren zur Herstellung der Verbindung der Formel I, wie in Anspruch 1 definiert, aus einer Verbindung der Formel II

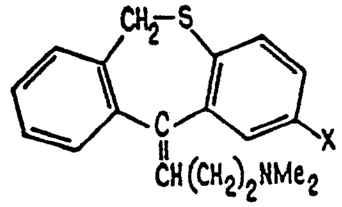

II

a) wenn X eine austretende Gruppe, beispielsweise Chlor, Brom oder Jod bedeutet, durch Reaktion mit Kupfercyanid,

b) wenn X eine Jodgruppe bedeutet, durch Umsetzung mit Kaliumcyanid in Gegenwart von Tetrakis(triphenylphosphin)palladium(O),

c) wenn X eine Amidgruppe der Formel —$CONH_2$ bedeutet, durch Dehydrierung mit, beispielsweise Phosphorylchloridtriphenylphosphin in Tetrachlormethan oder p-Toluolsulphonylmethylisocyanid,

d) wenn X eine Gruppe der Formel —CH=NOH bedeutet, durch Dehydrierung mit, beispielsweise Phosphorylchlorid, Dicyclohexylcarbodimid in Gegenwart von Kupferionen oder 1,1'-Carbonyldiimidazol,

e) wenn X einen $NO_2$-Gruppe bedeutet, durch Reduzierung (beispielsweise durch Zinnchlorid), um eine Amingruppe zu erhalten, die man in eine Cyanogruppe mittels der Sandmeyer-Reaktion überführt.

5. Verfahren zur Herstellung der Verbindung der Formel I, wie in Anspruch 1 definiert, indem man eine Verbindung der Formel (III)

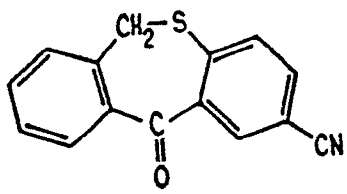

III

mit einem Reagenz der Formel $Ph_3P{=}CH(CH_2)_2R$ einsetzt, worin R $NMe_2$ oder eine Gruppe, beispielsweise Halogengruppe, die durch $NMe_2$ ersetzt werden kann, bedeutet.

6. Verfahren zur Herstellung der Verbindungen der Formel I, wie in Anspruch 1 definiert, durch Umsetzung der Carbonsäure der Formel IV

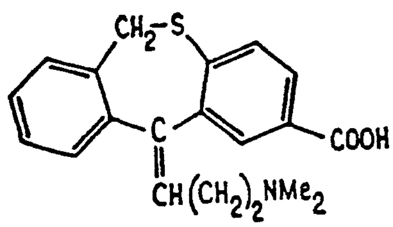

IV

a) mit Benzolsulphonamid oder

b) mit Harnstoff in Gegenwart von Sulphaminsäure.

**Revendications**

1. 11-(3-Diméthylaminopropylidène)-6,11-dihydrodibenzo(b,e)thiépine-2-carbonitrile de la formule I

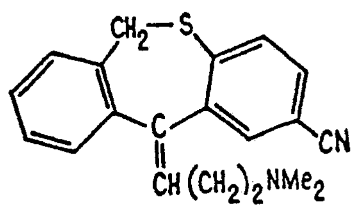

I.

et ses sels pharmaceutiquement acceptables.

2. Bromhydrate de 11-(3-diméthylaminopropylidène)-6,11-dihydrodibenzo(b,e)thiépine-2-carbonitrile.

3. Compositions pharmaceutiques contenant une quantité thérapeutiquement efficace des composés suivant la revendication 1 ou la revendication 2, en même temps qu'un véhicule, excipient ou diluant pharmaceutiquement acceptable.

4. Procédé de préparation du composé de la formule I, suivant la revendication 1, à partir d'un composé de la formule II

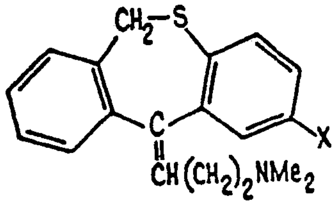

II

a) par réaction sur le cyanure cuivreux lorsque X représente un groupe labile, par exemple un atome de chlore, de brome ou d'iode,

b) par réaction sur le cyanure de potassium en présence de tétrakis(triphénylphosphine) palladium (O) lorsque X représente un atome d'iode,

c) par déshydratation réalisée à l'aide de, par exemple, le chlorure de phosphoryle, la triphénylphosphine dans le tétrachlorométhane, ou l'isocyanure de p-toluéne-sulfonylméthyle lorsque X représente un groupe amide de la formule $—CONH_2$.

d) par déshydratation à l'aide de, par exemple, le chlorure de phosphoryle, le dicyclohexylcarbodiimide en présence d'ions cuivriques, ou le 1,1'-carbonyldiimidazole, lorsque X représente un groupe de la formule —CH=NOH,

e) par réduction (par exemple à l'aide de chlorure stanneux) lorsque X représente un radical $NO_2$, de façon à obtenir un radical amine que l'on convertit en un radical cyano par l'intermédiaire d'une réaction de Sandmeyer.

5. Procédé de préparation du composé de la formule I suivant la revendication 1, caractérisé en ce que l'on fait réagir un composé de la formule III

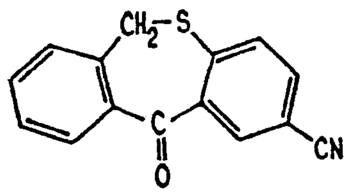

III

sur un réactif de la formule $Ph_3P=CH(CH_2)_2R$ dans laquelle R représente le radical $NMe_2$ ou un substituant tel qu'un atome d'halogène que l'on peut remplacer par le radical $NMe_2$.

6. Procédé de préparation du composé de la formule I suivant la revendication 1, caractérisé en ce que l'on fait réagir l'acide carboxylique de la formule IV

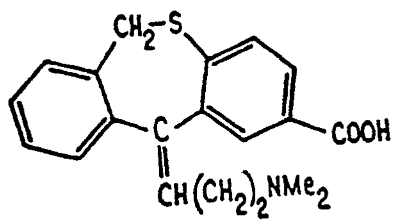

IV

a) sur le benzènesulfonamide, ou
b) sur l'urée en présence d'acide sulfamique.

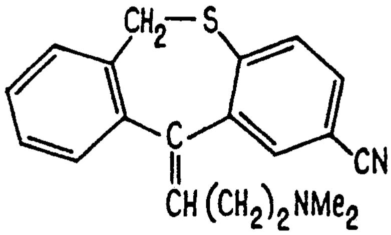

I

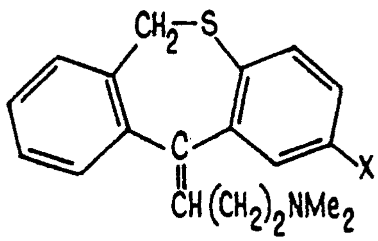

II

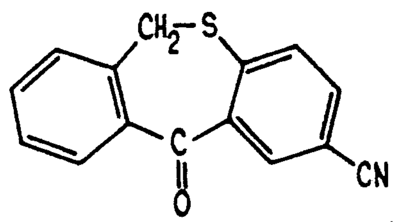

III

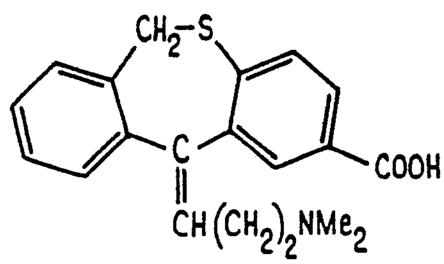

IV

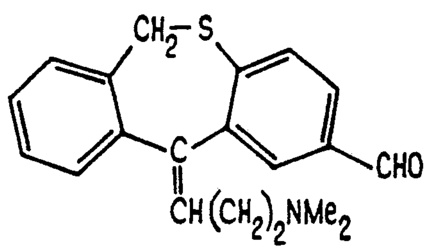

V

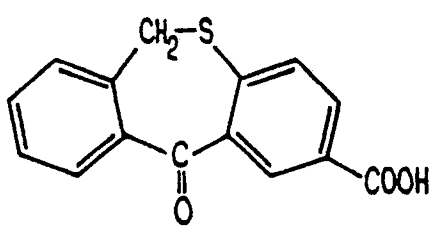

VI